# EUROPEAN PATENT SPECIFICATION

(11) **EP 4 213 784 B1**
(45) Date of publication and mention of the grant of the patent: **30.07.2025**
(21) Application number: 21790562.9
(22) Date of filing: 23.09.2021
(51) Int. Cl.: A61F 13/08, A61F 13/00

(54) **LYMPHEDEMA AND SURGICAL WOUND DRESSING**
LYMPHÖDEM UND CHIRURGISCHER WUNDVERBAND
LYMPHOEDÈME ET PANSEMENT CHIRURGICAL

(30) Priority: 21.10.2020 US 202063094630 P
(43) Date of publication of application: 26.07.2023
(73) Proprietor: KCI Manufacturing Unlimited Company, Athlone, Co. Westmeath, N37XF22 (IE)
(72) Inventor: LOCKE, Christopher Brian, Athlone, Co. Westmeath, N37XF22 (IE); PRATT, Benjamin Andrew, Athlone, Co. Westmeath, N37XF22 (IE)
(74) Representative: Simmons & Simmons
(86) International application number: PCT/IB2021/058701
(87) International publication number: WO 2022/084770

(56) References cited:
- US-A1- 2019 111 298
- US-A1- 2020 000 643
- US-B2- 6 554 786

## Description

### TECHNICAL FIELD

The invention set forth in the appended claims relates generally to tissue treatment apparatuses, systems, more particularly, but without limitation, to apparatuses, systems, for treating tissue sites that include sprains and strains to subcutaneous tissue sites such as, for example, a ligament or muscle.

### BACKGROUND

A sprain is an injury resulting from the wrenching or twisting of a ligament or muscle of a joint, such as a knee or ankle, characterized by clinical symptoms including swelling, bruising or contusions, pain, and disablement of the joint. A sprain may further be characterized by edema which is an abnormal accumulation of fluid in cells, tissues, or cavities of the body resulting in swelling. Strains are sprains caused by exertion or an acute trauma event. These trauma events can include, for example, an abnormal muscle contraction, a high amount of specifically applied tension, or forced stretching of the muscle of the ligament. These injuries can be extremely debilitating, especially to professional and amateur athletes who can no longer participate in physical activities. In addition, the affected area, most commonly extremities such as the foot, ankle and knee, suffer from reduced range of motion.

Acute inflammation is a response to any type of trauma including trauma events causing a sprain or strain wherein the inflammation protects the tissue and removes any damaged material or tissue from the body. Enzymatic signaling agents including histamine, serotonin, bradykinin, and prostaglandin are normally released as part of the inflammatory process. These agents increase capillary membrane permeability in order to enhance the inflammatory process, but also result in edema from fluid accumulation during the interstitial phase. The signaling agents, therefore, cause the primary symptoms of inflammation: swelling, heat, redness and pain. This initial phase of inflammation can start after one or two days and end after three or four days. In some cases, the damage to the ligament can be even more severe. For example, high ankle sprains involve injury to the ligament above the ankle that joins together the tibia and fibula, or syndesmotic ligament. Regardless of the type of strain or sprain, a single injury has been shown to place the affected extremity at significantly greater risk of re-injury even after the first injury has healed.
US2020/0000643 discloses systems and methods for applying negative pressure to a tissue site, including a dressing with a manifold and a polymer film contact layer, featuring fluid restrictions that respond to pressure gradients.
US2019/0111298 discloses a belt designed for BFR systems, featuring an inflatable chamber within hermetically sealed materials, a gas input port, fastening mechanisms for secure fit, and a pre-tensioning system for initial compression.
US6,554,786 discloses a compression dressing for lymphoedema treatment, allowing self-application by patients. It features an elastically stretchable material with a loop pile structure, a separable slide fastener, and fabric tabs for secure attachment.

### BRIEF SUMMARY

The invention is defined by the independent claim. A selection of optional features of the invention is set out in the dependent claims.

Insofar as the term embodiment is used in the following, or features are presented as being optional, this should be interpreted in such a way that the only protection sought is that of the invention claimed (with due regard to Article 69 EPC and the protocol thereto).
References to "embodiment(s)" throughout the description which are not under the scope of the appended claims merely represent possible exemplary executions and are not part of the present invention.

### BRIEF DESCRIPTION OF THE DRAWINGS

Figure 1 is an isometric view of an example embodiment of a dressing that can provide therapy to a tissue site in accordance with this specification;
Figure 2 is an isometric view of another example embodiment of a dressing;
Figure 3 is a section view of the dressing of Figure 2 along line 3-3;
Figure 4A is an isometric view of another example embodiment of a dressing;
Figure 4B is an isometric detail view, with a portion shown in cross-section, of the dressing of Figure 4A;
Figure 5A is a section view of the dressing of Figure 4A and Figure 4B coupled to an epidermis of a patient proximate a tissue site and encircling the tissue site; and
Figure 5B is a section view of the dressing of Figure 4A and Figure 4B coupled to an epidermis of a patient proximate a tissue site and encircling the tissue site, wherein the dressing is applying an outward radial force on the tissue site.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

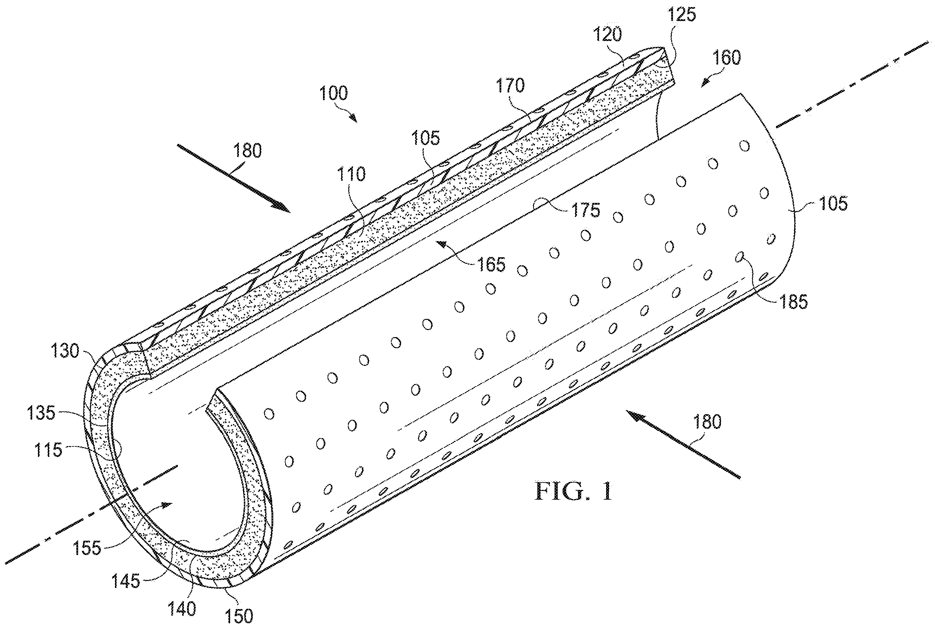

Figure 1 is an isometric view of an example embodiment of a dressing 100 that can provide therapy to a tissue site in accordance with this specification. The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. The dressing 100 described herein may be used to treat tissue sites that have intact skin or epidermis but include sprains and strains to subcutaneous tissue such as, for example, a ligament or a muscle.

As shown in Figure 1, the dressing 100 may include a first layer, such as a biasing or spring element 105, a second layer, such as a conformable layer 110, and a third layer, such as an adhesive layer 115. The biasing element 105, the conformable layer 110, and the adhesive layer 115 may be configured to at least partially encircle a tissue site. The biasing element 105 may have an unloaded state and a loaded state, and may be deflected from the unloaded state to the loaded stated. The dressing 100 may be coupled to a tissue site and may be configured to exert radial expansion forces on the tissue site.

The conformable layer 110 may be coupled to the biasing element 105 and the adhesive layer 115 may be coupled to the conformable layer 110 opposite the biasing element 105. Coupling may include fluid, mechanical, thermal, electrical, or chemical coupling (such as a chemical bond), or some combination of coupling in some contexts. In some embodiments, components may also be coupled by virtue of physical proximity, being integral to a single structure, or being formed from the same piece of material. In some embodiments, the edges of one or more of the biasing element 105, the conformable layer 110, and the adhesive layer 115 may be congruent, so that adjacent faces of the biasing element 105, the conformable layer 110, and the adhesive layer 115 are substantially coextensive and have substantially the same surface area. If the dressing 100 is applied to the tissue site, the conformable layer 110 may be between the tissue site and the biasing element 105.

The biasing element 105 may have a first side 120 and a second, patient-facing side 125. The conformable layer 110 may have a first side 130 and a second, patient-facing side 135. The adhesive layer 115 may have a first side 140 and a second, patient-facing side 145. The first side 130 of the conformable layer 110 may be coupled to the second, patient-facing side 125 of the biasing element 105. The first side 140 of the adhesive layer 115 may be coupled to the second, patient-facing side 135 of the conformable layer 110.

In some embodiments, the biasing element 105 may define the shape of the other components of the dressing 100, such as the conformable layer 110 and the adhesive layer 115, as well as the dressing 100 as a whole. In some embodiments, the biasing element 105 may include or be formed of a curved wall 150 forming a tubular shape. The biasing element 105 may further include a first open end 155, a second open end 160, and an opening 165 in the curved wall 150 extending from the first open end 155 to the second open end 160. The opening 165 may define a first edge 170 and a second edge 175. The dressing 100 may be sized such that, when the biasing element 105 is in the unloaded state, the anatomy proximate to the tissue site can be inserted into one of the first open end 155 or the second open end 160. For example, if the tissue site is located at or proximate to a wrist of a patient, the dressing 100 may be sized so that the dressing 100 can be slipped over the wrist. In some embodiments, when the dressing 100 is in the unloaded state, the dressing 100 may have a shape similar to the anatomy to which it is configured to be coupled to in its loaded state. For example, the dressing 100 may have a three-dimensional anatomical shape similar to a knee, ankle, foot, or wrist in its unloaded state. In some embodiments, the dressing 100 may be packaged, shipped, and/or sold having a three-dimensional anatomical shape in its unloaded state.

Compression of the biasing element 105, for example in the direction of arrows 180, from the unloaded state to the loaded state may bring the first edge 170 closer to the second edge 175. The biasing element 105 may be biased toward the unloaded state. Thus, when deflected to the loaded state, the biasing element 105 will want to return to the unloaded state. For example, when compressed, the biasing element 105 will want to move the first edge 170 and the second edge 175 away from one another.

In some embodiments, the biasing element 105 may include or be formed of a material that can be deflected from an unloaded state to a loaded state. For example, the biasing element 105 may be formed from one or more of the following materials: plastics, such as polypropylene (PP), acrylonitrile butadiene styrene (ABS), and polyvinyl chloride (PVC); metals, such as steel or alloys thereof; and/or composite materials. The biasing element 105 may have a thickness in a range of about 1 millimeter to about 3 millimeters. In some embodiments, the biasing element 105 may have a thickness less than 1 millimeter. In some embodiments, the biasing element 105 may have a thickness greater than 3 millimeters. In some embodiments, the thickness of the biasing element 105 may be constant across the biasing element 105. In some embodiments, the thickness of the biasing element 105 may vary across the biasing element 105.

As further shown in Figure 1, the biasing element 105 may include one or more apertures 185. The apertures 185 may be formed by cutting, perforating, punching, or by other suitable techniques for forming an aperture, opening, perforation, or hole in the biasing element 105, including but not limited to using a single- or multiple-blade cutter, a laser, a water jet, a hot knife, a computer numeric control (CNC) cutter, a hot wire, local RF or ultrasonic energy, and/or a single- or multiple-punch tool. In some embodiments, the apertures 185 may be molded into the biasing element 105, for example, in an injection molding process. The apertures 185 extend from the first side 120 to the second, patient-facing side 125 of the biasing element 105, creating a through hole or passage in the biasing element 105. The apertures 185 in the biasing element 105 may have many shapes, for example, including but not limited to circles, squares, stars, ovals, hexagons, polygons, slits, complex curves, rectilinear shapes, triangles or may have some combination of such shapes.

Each of the apertures 185 may have uniform or similar geometric properties. For example, in some embodiments, each of the apertures 185 may be circular apertures, having substantially the same diameter. In some embodiments, each of the apertures 185 may have a diameter in a range of about 1 millimeter to about 50 millimeters. In other embodiments, each of the apertures 185 may have a diameter in a range of about 1 millimeter to about 20 millimeters. In other embodiments, each of the apertures 185 may have a diameter in a range of about 1 millimeter to about 5 millimeters. In yet other embodiments, each of the apertures 185 may have a diameter in a range of about 2 millimeters to about 3 millimeters.

The conformable layer 110 may be a material configured to conform to the shape of the tissue site. The conformable layer 110 may increase the comfort of the dressing 100. In some embodiments, the conformable layer 110 may be vapor permeable. In some embodiments, the conformable layer 110 may be a manifold, which may include a plurality of pathways, which can be interconnected to improve distribution or collection of fluids. In some illustrative embodiments, a manifold may include or be formed of a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively include projections that form interconnected fluid pathways. For example, a manifold may be molded to provide surface projections that define interconnected fluid pathways.

In some embodiments, the conformable layer 110 may include or be formed of reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the conformable layer 110 may also vary according to needs of a prescribed therapy. The 25% compression load deflection of the conformable layer 110 may be at least 0.35 pounds per square inch, and the 65% compression load deflection may be at least 0.43 pounds per square inch. In some embodiments, the tensile strength of the conformable layer 110 may be at least 10 pounds per square inch. The conformable layer 110 may have a tear strength of at least 2.5 pounds per inch. In some embodiments, the conformable layer 110 may have a tear strength of at least 20 N. In some embodiments, the conformable layer 110 may be foam formed of polyols such as polyester or polyether, isocyanate such as toluene diisocyanate, and polymerization modifiers such as amines and tin compounds. In some examples, the conformable layer 110 may be reticulated polyurethane foam such as found in GRANUFOAM^{™} dressing or V.A.C. VERAFLO^{™} dressing, both available from Kinetic Concepts, Inc. of San Antonio, Texas.

The thickness of the conformable layer 110 may also vary according to needs of a prescribed therapy. The thickness of the conformable layer 110 can also affect the conformability of the conformable layer 110. In some embodiments, a thickness in a range of about 2 millimeters to 4 millimeters may be suitable. In some embodiments, the conformable layer 110 may have a thickness less than 2 millimeters. In some embodiments, the conformable layer 110 may have a thickness greater than 4 millimeters.

The conformable layer 110 may be either hydrophobic or hydrophilic. In an example in which the conformable layer 110 may be hydrophilic, the conformable layer 110 may also wick fluid away from a tissue site. The wicking properties of the conformable layer 110 may draw fluid away from a tissue site by capillary flow or other wicking mechanisms. The fluid drawn away may flow out of or evaporate through the apertures 185 of the biasing element 105. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the conformable layer 110 may include or be formed of a closed-cell foam. For example, the conformable layer 110 may be formed of silicone, polyurethane (PU), or ethylene vinyl acetate (EVA). The structure of these closed-cell foams can provide a surface that interacts little, if any, with biological tissues and fluids, providing a surface that may encourage the free flow of liquids and low adherence, which can be particularly advantageous for many applications. For example, the conformable layer 110 may be a closed-cell foam having an average pore size in a range of about 0.2 millimeters (200 microns) to about 1 millimeter (1000 microns). In some embodiments, the conformable layer 110 may be a closed-cell foam having a porosity in a range of about 200 ppi to about 30 ppi. In some examples, the conformable layer 110 may be a closed-cell foam available from Zotefoams plc of Croydon, United Kingdom.

Additionally, in some embodiments, the conformable layer 110 may be thermally conductive. The conformable layer 110 may be configured to remove heat from the tissue site to reduce thermal build-up at the tissue. This may increase comfort of the dressing 100. In some embodiments, the conformable layer 110 may be metal loaded to increase thermal conductivity.

In some embodiments, the conformable layer 110 may store, or immobilize, liquid from a tissue site. The conformable layer 110 may contain any substance capable of storing a liquid. The conformable layer 110 may include, without limitation, super absorbent fiber/particulates, hydrofibre, sodium carboxymethyl cellulose, and/or alginates. In some exemplary embodiments, the conformable layer 110 may include a superabsorbent polymer (SAP). Generally, relative to their mass, SAPs can absorb and retain large quantities of liquid, and in particular water. SAPs may be used to hold and stabilize or solidify wound fluids. SAPs may be of the type often referred to as "hydrogels," "super-absorbents," or "hydrocolloids." In some embodiments, the conformable layer 110 may include SAP fibers or spheres. The SAP fibers may be either woven or non-woven. In some embodiments, the SAPs may be dispersed as pellets throughout and/or embedded as a sheet-like layer within the conformable layer 110.

The SAPs may be formed in several ways, for example, by gel polymerization, solution polymerization, or suspension polymerization. Gel polymerization may involve blending of acrylic acid, water, cross-linking agents, and ultraviolet (UV) initiator chemicals. The blended mixture may be placed into a reactor where the mixture is exposed to UV light to cause crosslinking reactions that form the SAP. The mixture may be dried and shredded before subsequent packaging and/or distribution. Solution polymerization may involve a water based monomer solution that produces a mass of reactant polymerized gel. The monomer solution may undergo an exothermic reaction that drives the crosslinking of the monomers. Following the crosslinking process, the reactant polymer gel may be chopped, dried, and ground to its final granule size. Suspension polymerization may involve a water-based reactant suspended in a hydrocarbon-based solvent. However, the suspension polymerization process must be tightly controlled and is not often used.

SAPs absorb liquids by bonding with water molecules through hydrogen bonding. Hydrogen bonding involves the interaction of a polar hydrogen atom with an electronegative atom. As a result, SAPs absorb water based on the ability of the hydrogen atoms in each water molecule to bond with the hydrophilic polymers of the SAP having electronegative ionic components. High absorbing SAPs are formed from ionic crosslinked hydrophilic polymers such as acrylics and acrylamides in the form of salts or free acids. Because the SAPs are ionic, they are affected by the soluble ionic components within the solution being absorbed and will, for example, absorb less saline than pure water. The lower absorption rate of saline is caused by the sodium and chloride ions blocking some of the water absorbing sites on the SAPs. If the fluid being absorbed by the SAP is a solution containing dissolved mineral ions, fewer hydrogen atoms of the water molecules in the solution may be free to bond with the SAP. Thus, the ability of an SAP to absorb and retain a fluid may be dependent upon the ionic concentration of the fluid being absorbed. For example, an SAP may absorb and retain de-ionized water up to 500 times the weight of the dry SAP. In volumetric terms, an SAP may absorb fluid volumes as high as 30 to 60 times the dry volume of the SAP. Other fluids having a higher ionic concentration may be absorbed at lower quantities. For example, an SAP may only absorb and retain a solution that is 0.9% salt (NaCl) up to 50 times the weight of the dry SAP.

In some embodiments, the conformable layer 110 may include or be formed of a KERRAMAX CARE^{™} Super-Absorbent Dressing material available from Kinetic Concepts, Inc. of San Antonio, Texas. For example, the conformable layer 110 may include or be formed of a superabsorbent laminate comprised of 304 g.s.m. FAVOR-PAC^{™} 230 superabsorbent powder glued by PAFRA^{™} 8667 adhesive between two layers of 50 g.s.m. LIDRO^{™} non-woven material. In some embodiments, the conformable layer 110 may include or be formed of an absorbent available from Gelok International.

Because the dressing 100 may be positioned on the tissue site for a prolonged period of time, the conformable layer 110 may also possess an antimicrobial property to mitigate the risk of fungal infection and the spread of such infections caused by perspiration and warm temperatures in the dressing 100. The antimicrobial property of the conformable layer 110 may reduce the effect of VOCs to reduce odors being generated by the dressing 100. The antimicrobial property may be achieved by means of a silver coating that covers the conformable layer 110 or by a silver additive to the conformable layer 110. In some embodiments, the conformable layer 110 may include activated charcoal to reduce or eliminate odor. For example, the conformable layer 110 may be loaded with activated charcoal particles throughout its thickness. In some embodiments, the conformable layer 110 may be coated with activated charcoal. In addition to reducing odor, the activated charcoal may also increase evaporation rates from the dressing 100 as fluid molecules may be drawn to the conformable layer 110. In some embodiments, the conformable layer 110 may include or be coated with oxysalts, which can reduce bacterial colonization within the conformable layer 110.

The adhesive layer 115 is configured to be coupled to the tissue site. The dressing 100 thus may be coupled to the tissue site by the adhesive layer 115. For example, the second, patient-facing side 145 of the adhesive layer 115 is configured to contact the tissue site. The adhesive layer 115 may include or be formed of an adhesive. The adhesive may be coupled to the second, patient-facing side 135 of the conformable layer 110. In some embodiments, the adhesive may be coated, printed, or deposited on the second, patient-facing side 135 of the conformable layer 110. The adhesive may be a medically-acceptable adhesive. The adhesive may also be flowable. For example, the adhesive may be an acrylic adhesive, rubber adhesive, high-tack or tacky silicone adhesive, polyurethane, or other adhesive substance. In some embodiments, the adhesive may be a pressure-sensitive adhesive, such as an acrylic adhesive with coating weight of 15 grams/m² (gsm) to 70 grams/m² (gsm). The adhesive layer 115 may be capable of resisting the expansion forces of the biasing element 105, such that as the biasing element 105 applies a pulling force on the tissue site, the adhesive layer 115 does not release from the tissue site. To achieve the desired bond to the tissue site, the adhesive layer 115 may be dependent upon the surface area of the adhesive of the adhesive layer 115 and the peel strength of the adhesive. Having a high surface area may be desired as a larger adhesive surface area may tend to distribute the pulling force from the biasing element 105 on a larger area of the tissue site, whereas a smaller adhesive surface area may result in skin tearing and or redness. In some embodiments, the adhesive may have a peel strength or resistance to being peeled from a stainless steel material in a range of about 5 N to about 20 N. In some embodiments, the adhesive may have a peel strength or resistance to being peeled from a stainless steel material of about 10 N. The peel strength may be measured by applying a 1 inch (2.54 cm) wide test strip of the adhesive to a stainless steel plate using a roller. The test strip is then peeled back over itself (at an angle of 180 degrees) and the force required to peel the test strip is measured. The test is conducted at on a stainless steel substrate at 23 degrees C at 50 % relative humidity based on ASTM D3330.

In some embodiments, the adhesive of the adhesive layer 115 may be reduced or deactivated using ultraviolet light. For example, the adhesive of the adhesive layer 115 may be an ultraviolet switching adhesive. Ultraviolet light may be shined upon the dressing 100 and the ultraviolet light may reduce the peel strength of the adhesive a sufficient amount to allow removal of the dressing 100 from the tissue site without damage to the tissue site. Thicker adhesives, or combinations of adhesives, may be applied in some embodiments to improve the adhesion to the tissue site. Other example embodiments of an adhesive may include a double-sided tape, paste, hydrocolloid, hydrogel, silicone gel, or organogel.

Individual components of the dressing 100 may be bonded or otherwise secured to one another with a solvent or non-solvent adhesive, or with thermal welding, for example, without adversely affecting fluid management.

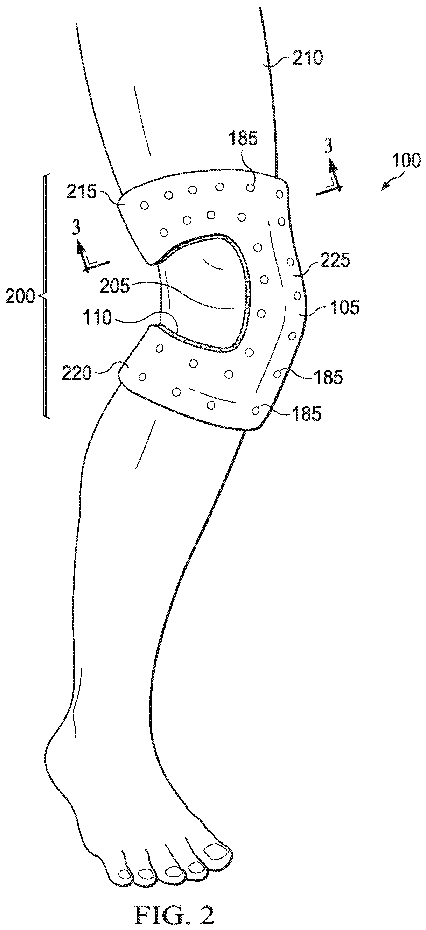

Figure 2 is an isometric view of another example embodiment of a dressing 100. In the example embodiment of Figure 2, the dressing 100 is configured for delivering therapy to a tissue site 200, such as, for example, proximate a knee 205 of a patient. The dressing 100 may be configured to be disposed at least partially around the tissue site 200. In some embodiments, the dressing 100, in its unloaded state, may be shaped in three dimensions and may have contours and/or a variable thickness to provide appropriate expansion over the tissue site 200. The dressing 100 may have an anatomical shape in some embodiments, or may be shaped such that when wrapped around the patient it is anatomically shaped or conforms to an anatomical shape. For example, the dressing 100 has a shape configured to conform to at least a portion of the knee 205 and the leg 210.

In the example shown in Figure 2, some embodiments of the biasing element 105 may include a first cuff 215, a second cuff 220, and a stem 225 connected to and extending between the first cuff 215 and the second cuff 220. The conformable layer 110 and the adhesive layer 115 may be coextensive with the biasing element 105 or similarly shaped, such that the conformable layer 110 and the adhesive layer 115 may also include a first cuff, a second cuff, and a stem by way of analogy. Thus, the dressing 100, the conformable layer 110, and the adhesive layer 115 can each interchangeably be referred to as including the first cuff 215, the second cuff 220, and the stem 225 connected to and extending between the first cuff 215 and the second cuff 220.

The first cuff 215 may be configured to extend at least partially around the back of the leg 210 above the knee 205 and the second cuff 220 may be configured to extend at least partially around the back of the leg 210 below the knee 205. The stem 225 may be configured to cover at least a portion of the front of the knee 205. Additionally, in some embodiments, the stem 225 does not extend around the back of the knee 205, leaving the popliteal fossa region of the knee 205 uncovered by the dressing 100. As further shown in Figure 2, in some embodiments, the some of the apertures 185 in the biasing element 105 may be located in one or more of the first cuff 215, the second cuff 220, and the stem 225.

Although shown in the example embodiment of Figure 2 as being used to treat a tissue site 200 proximate a knee 205, some embodiments of the dressing 100 may be configured for treating other portions of a patient. Other exemplary embodiments of the dressing 100 may be suitable for the treatment of ligaments or muscles associated with other joints such as, for example, a knee, ankle, wrist, shoulder, finger, hip, or elbow joint.

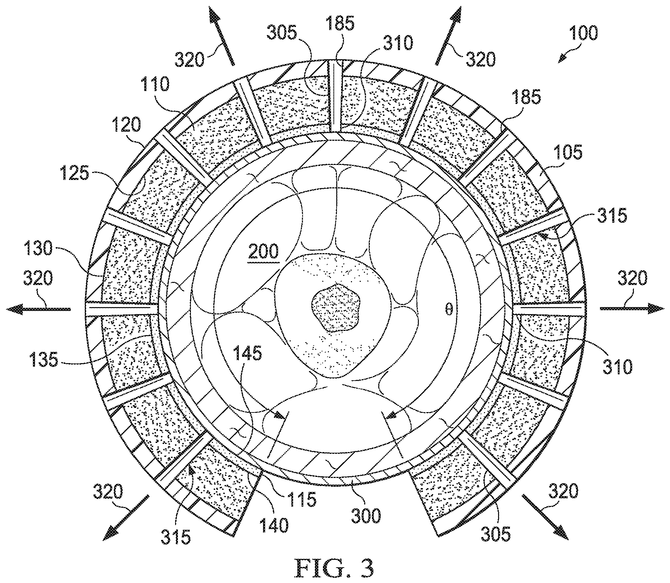

Figure 3 is a section view of the dressing 100 of Figure 2 along line 3-3. As shown in Figure 3, the dressing 100 is shown as coupled to the epidermis 300 of a patient proximate the tissue site 200 and at least partially encircling the tissue site 200. The biasing element 105 may have an arc-shaped cross-section having a central angle Θ of at least 180 degrees when the biasing element 105 is in the unloaded state. In some embodiments, the biasing element 105 may have an arc-shaped cross-section having a central angle Θ of at least 270 degrees when the biasing element 105 is in the unloaded state. Because the biasing element 105 may define the shape of the other components of the dressing 100, such as the conformable layer 110 and the adhesive layer 115, as well as the dressing 100 as a whole, the conformable layer 110, the adhesive layer 115, and the dressing 100 may also have an arc-shaped cross-section having a central angle Θ of at least 180 degrees when the biasing element 105 is in the unloaded state. In some embodiments, the conformable layer 110, the adhesive layer 115, and the dressing 100 as a whole may also have an arc-shaped cross-section having a central angle Θ of at least 270 degrees when the biasing element 105 is in the unloaded state.

As further shown in Figure 3, the conformable layer 110 may have one or more apertures 305. The apertures 305 may be formed by cutting, perforating, punching, or by other suitable techniques for forming an aperture, opening, perforation, or hole in the conformable layer 110, including but not limited to using a single- or multiple-blade cutter, a laser, a water jet, a hot knife, a computer numeric control (CNC) cutter, a hot wire, local RF or ultrasonic energy, and/or a single- or multiple-punch tool. The apertures 305 extend from the first side 130 to the second, patient-facing side 135 of the conformable layer 110, creating a through hole or passage in the conformable layer 110. The apertures 305 in the conformable layer 110 may have many shapes, for example, including but not limited to circles, squares, stars, ovals, hexagons, polygons, slits, complex curves, rectilinear shapes, triangles or may have some combination of such shapes.

Each of the apertures 305 may have uniform or similar geometric properties. For example, in some embodiments, each of the apertures 305 may be circular apertures, having substantially the same diameter. In some embodiments, each of the apertures 305 may have a diameter in a range of about 1 millimeter to about 50 millimeters. In other embodiments, each of the apertures 305 may have a diameter in a range of about 1 millimeter to about 20 millimeters. In other embodiments, each of the apertures 305 may have a diameter in a range of about 1 millimeter to about 5 millimeters. In yet other embodiments, each of the apertures 305 may have a diameter in a range of about 2 millimeters to about 3 millimeters.

In some embodiments, the adhesive layer 115 may be continuous or discontinuous. Discontinuities in adhesive layer 115 may be provided by one or more apertures 310 in the adhesive layer 115. The apertures 310 in the adhesive layer 115 may be formed after application of the adhesive layer 115 or by coating the adhesive layer 115 in patterns on a carrier layer, such as, for example, the second, patient-facing side 135 of the conformable layer 110. The apertures 310 may be formed by cutting, perforating, punching, or by other suitable techniques for forming an aperture, opening, perforation, or hole in the adhesive layer 115, including but not limited to using a single- or multiple-blade cutter, a laser, a water jet, a hot knife, a computer numeric control (CNC) cutter, a hot wire, local RF or ultrasonic energy, and/or a single- or multiple-punch tool. The apertures 310 extend from the first side 140 to the second, patient-facing side 145 of the adhesive layer 115, creating a through hole or passage in the adhesive layer 115. The apertures 310 in the adhesive layer 115 may have many shapes, for example, including but not limited to circles, squares, stars, ovals, hexagons, polygons, slits, complex curves, rectilinear shapes, triangles or may have some combination of such shapes.

Each of the apertures 310 may have uniform or similar geometric properties. For example, in some embodiments, each of the apertures 310 may be circular apertures, having substantially the same diameter. In some embodiments, each of the apertures 310 may have a diameter in a range of about 1 millimeter to about 50 millimeters. In other embodiments, each of the apertures 310 may have a diameter in a range of about 1 millimeter to about 20 millimeters. In other embodiments, each of the apertures 310 may have a diameter in a range of about 1 millimeter to about 5 millimeters. In yet other embodiments, each of the apertures 310 may have a diameter in a range of about 2 millimeters to about 3 millimeters.

As illustrated in Figure 3, the apertures 310 in the adhesive layer 115 may be aligned, overlapping, in registration with, or otherwise fluidly coupled to the apertures 305 in the conformable layer 110 and the apertures 185 in the biasing element 105 in some embodiments. Thus, at least some of the plurality of apertures 310 have corresponding apertures 305 and apertures 185, wherein the corresponding apertures 185, apertures 305, and apertures 310 are in fluid communication. The corresponding apertures 185, apertures 305, and apertures 310 may cooperate to form one or more passageways 315 through which fluid may flow. The apertures 185, apertures 305, and apertures 310, and the passageways 315 formed thereby, may enhance the MVTR of the dressing 100 in some example embodiments, allowing skin moisture, perspiration, or other fluids to migrate away from the patient through the dressing 100.

In some embodiments, the apertures 185, apertures 305, and apertures 310, and the passageways 315 formed thereby, may allow for fluids to be supplied to the tissue site 200. For example, over time, the bond of the adhesive layer 115 to the tissue site may increase, and thus the adhesive layer 115 may offer higher resistance to removal. Additionally, the application of heat (such as heat from the patient) can increase the bond strength of the adhesive layer 115. Accordingly, the passageways 315 may be configured to permit a liquid to be drawn through the passageways 315 such that the liquid contacts the adhesive layer 115. The liquid then interacts with the adhesive layer 115 to reduce the peel strength of the adhesive layer 115. This allows the adhesive layer 115 to be removed from the tissue site 200 without damage to the tissue site 200, even if the dressing 100 has been adhered to the tissue site for a long period of time. In some embodiments, the liquid may be an alcohol, such as isopropyl alcohol. For example, a user may apply a small amount of isopropyl alcohol to the dressing 100. The isopropyl alcohol may then be drawn through the passageways 315 and will soften the adhesive of the adhesive layer 115 over about a 2 to 3 minute period, thus reducing the peel strength of the adhesive of the adhesive layer 115. The dressing 100 may then be removed from the tissue site 200. After removal, the isopropyl alcohol will evaporate, and the peel strength of the adhesive of the adhesive layer 115 will return to only slightly less than its original level (about 80%), allowing the dressing 100 to be re-adhered to the tissue site 200, if desired.

In operation, the dressing 100 may be placed over, on, or otherwise proximate to the tissue site 200. In some embodiments, the tissue site 200 may be inserted into the dressing 100. The dressing 100, including one or more of the biasing element 105, the conformable layer 110, and the adhesive layer 115 may at least partially encircle the tissue site 200. Then, an external force may be placed on the biasing element 105 to place the biasing element 105 in the loaded state. For example, the dressing 100 may be compressed to deflect the biasing element 105 from the unloaded state to the loaded state. The external force placed on the biasing element 105 may be sufficient to couple the dressing 100 on, around, or otherwise proximate to the tissue site 200 using the adhesive layer 115. For example, the dressing 100 may be pressed onto the epidermis 300 so that the adhesive layer 115 is sufficiently adhered to the epidermis 300, such that when the external force is removed from the biasing element 105, the dressing 100 remains coupled to the epidermis 300. During application of the dressing 100, the conformable layer 110 may conform to the shape of the tissue site 200 and any anatomy surrounding the tissue site 200 to ensure contact between the adhesive layer 115 and the tissue site 200 across some or all of the surface area of the adhesive layer 115.

Because the biasing element 105 is configured to return to the unloaded state from the loaded state, the biasing element 105 exerts a pulling force on the tissue site 200. In some embodiments, the adhesive layer 115 has a peel strength that is at least 30% greater than the pulling force of the biasing element 105. This reduces or prevents the adhesive layer 115 from becoming detached from the tissue site 200 and any anatomy surrounding the tissue site 200.

As illustrated in the example of Figure 3, after the external force is removed from the biasing element 105, the spring force in the biasing element 105 that urges the biasing element 105 from the loaded state to the unloaded state, pulls the intact skin radially outwardly as shown by arrows 320. The outward force being distributed to the epidermis 300 by the biasing element 105 can promote perfusion by pulling the epidermis 300 outward for a sustained period of time rather than compressing the tissue site 200. The pulling force exerted by the biasing element 105 on the tissue site 200 can increase blood and lymphatic flow through the tissue site 200.

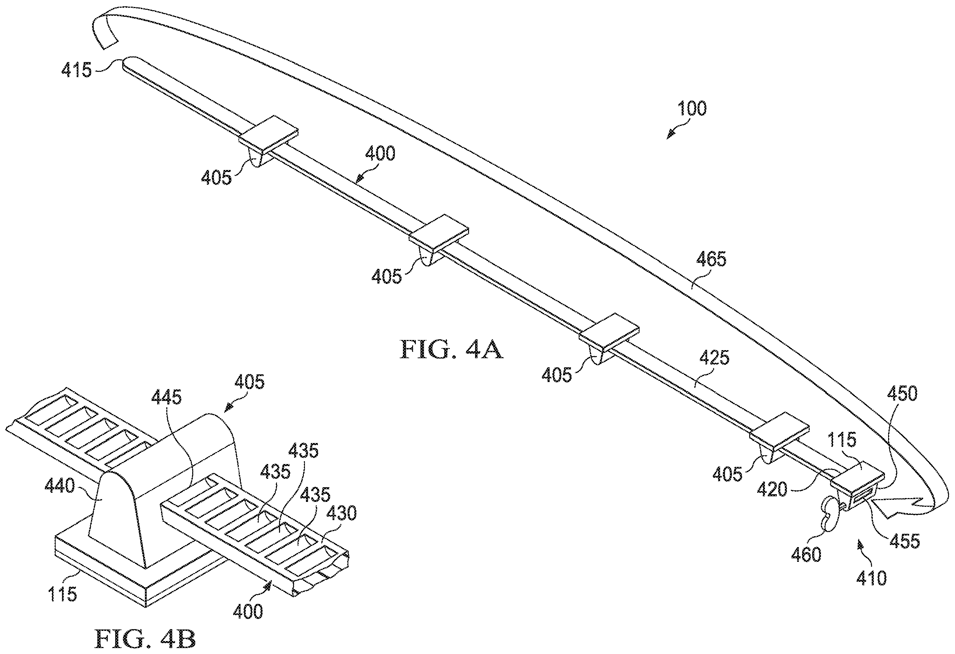

Figure 4A is an isometric view of another example embodiment of the dressing 100. Figure 4B is an isometric detail view, with a portion shown in cross-section, of the dressing 100 of Figure 4A. As shown in Figure 4A, some embodiments of the dressing 100 may include an elongate strap 400, one or more slider elements 405 coupled to the elongate strap 400, and a winder element 410 coupled to the elongate strap 400.

The elongate strap 400 may have a first end 415, a second end 420, and a length extending between the first end 415 and the second end 420. In some embodiments, the elongate strap 400 may have a rectangular cross-section having a first side 425 and a second side 430. The second side 430 may have a pattern of ratchet teeth 435 extending at least along a portion of the length of the elongate strap 400. The elongate strap 400 may be formed of a strip of metal or plastic, such as nylon. The elongate strap 400 is similar to a cable tie or zip tie. Although the dressing 100 is shown as having a single elongate strap 400, in some embodiments, the dressing 100 may include two or more elongate straps 400.

The slider elements 405 may include a slider body 440 having an aperture 445 that is configured to receive the elongate strap 400. For example, the aperture 445 may be sized and shaped to receive the elongate strap 400. The slider elements 405 may be on the elongate strap 400 and be configured to move or slide freely along the length of the elongate strap 400. The slider elements 405 may further include an adhesive layer 115 coupled to the slider body 440, wherein the adhesive layer 115 is configured to couple the slider elements 405 to the tissue site 200.

As shown in Figure 4A, the dressing 100 includes four slider elements 405. However, embodiments of the dressing 100 may include any number of slider elements 405. For example, fewer than four or greater than four slider elements 405 may be used depending on the therapy needs, the size of the tissue site 200, and/or the size of the anatomy proximate the tissue site 200. For example, more slider elements 405 may be used if the tissue site 200 is proximate the knee 205 and fewer slider elements 405 may be used if the tissue site 200 is proximate an elbow or wrist of a patient.

The winder element 410 may be coupled to the second end 420 of the elongate strap 400. For example, the winder element 410 may be fixed to the second end 420 such that there is no relative movement between the second end 420 and the winder element 410. The winder element 410 may include a winder body 450 and a winder mechanism (not shown). The winder body 450 may have an aperture 455 that is configured to receive the elongate strap 400. For example, the aperture 455 may be sized and shaped to receive the elongate strap 400. The winder mechanism may be configured to advance or retreat the elongate strap 400 through the aperture 455. The winder mechanism may be any suitable ratcheting device including, for example, a ratchet wheel and pawl operable on the elongate strap 400 to move the elongate strap 400 relative to the winder body 450. For example, the winder mechanism may be configured to engage the ratchet teeth 435 on the second side 430 of the elongate strap 400. The winder mechanism may include a toothed gear that can cooperate with the ratchet teeth 435. The winder mechanism may be coupled to a key 460, wherein rotation of the key 460 can cause rotation of the winder mechanism. The first end 415 of the elongate strap 400 is configured to be folded over and inserted into and through the aperture 455 of the winder body 450 as shown by arrow 465. The elongate strap 400 can be advanced into the aperture 455 of the winder body 450 by pushing or pulling it through the aperture 455. In some embodiments, the elongate strap 400 may only be retreated out of the aperture 455 by rotation of the key 460.

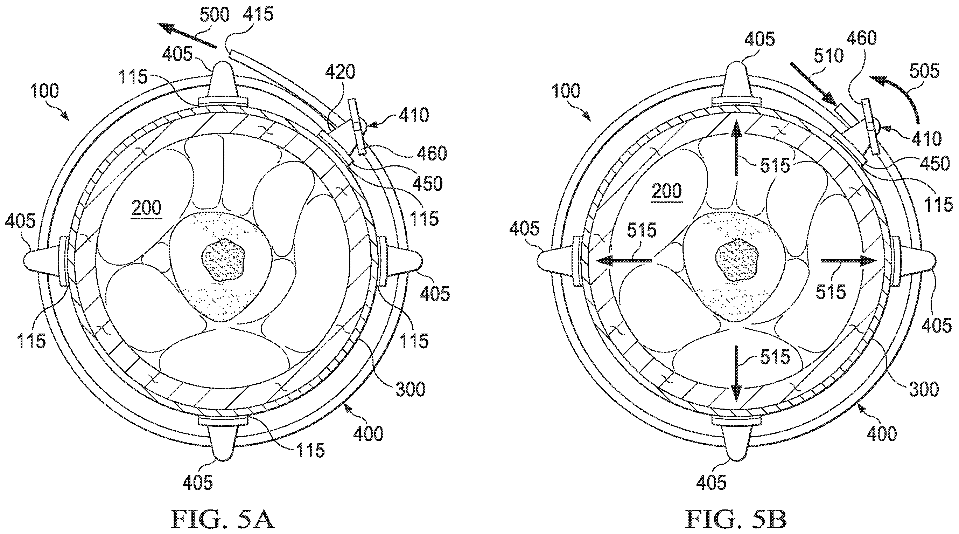

Figure 5A and Figure 5B are section views of the dressing 100 of Figure 4A and Figure 4B coupled to the epidermis 300 of a patient proximate the tissue site 200 and encircling the tissue site 200. In operation, the dressing 100 may be placed over, on, or otherwise proximate to the tissue site 200. The elongate strap 400 may be wrapped circumferentially around the tissue site 200. When wrapped around the tissue site 200, the dressing 100 may form a ring around the tissue site 200. The first end 415 of the elongate strap 400 may be extended through the aperture 455 (not shown) of the winder body 450. The slider elements 405 may be coupled to the epidermis 300 proximate the tissue site 200 using the adhesive layers 115. Any slack in the elongate strap 400 may be removed by advancing the first end 415 of the elongate strap 400 through the winder body 450 along arrow 500, effectively reducing the diameter of the ring formed by the dressing 100 around the tissue site 200. The first end 415 of the elongate strap 400 may be advanced, for example, by pulling on the elongate strap 400. Following application of the dressing 100 on the tissue site 200, the key 460 may be rotated, for example along arrow 505, to move the first end 415 of the elongate strap 400 toward to the winder body 450 along arrow 510. Moving the first end 415 of the elongate strap 400 increases the diameter of the ring formed by the dressing 100. Because the dressing 100 is adhered to the tissue site 200 by the adhesive layers 115 on the slider elements 405, increasing the diameter of the ring formed by the dressing 100 exerts a pulling force on the tissue site 200 as represented by arrows 515.

In some embodiments, key 460 can be removed from the dressing 100 after the desired tension is applied. This may reduce or eliminate the key 460 from snagging on clothing, medical equipment, other persons, or other objects. In some embodiments, the winder mechanism may be configured to slip on the ratchet teeth 435 of the elongate strap 400 or disengage from the ratchet teeth 435 if the tension applied by the key 460 exceeds a certain tension level. This may provide a safety mechanism so that too high a pulling force is not applied to the tissue site 200. In some embodiments, an electric motor may be coupled to the winder mechanism to move the elongate strap 400. A controller may control the electric motor in response to a sensed motor current draw and/or strain on the elongate strap 400 to ensure that the tension does not exceed a certain tension level. In some embodiments, the controller may be on board the dressing 100. In some embodiments, the electric motor may communicate with and/or controlled by a remote controller, either wired or wirelessly. The remote controller may be, for example, a smartphone.

The systems, apparatuses, and methods described herein may provide significant advantages. For example, use of the dressing 100 to treat wounds, strains, sprains, and other injuries to ankles and other joints can significantly reduce recovery time. The standard of care for strains and sprains for many decades has included rest, ice, compression and elevation. After a period of anywhere from 10 days to 24 weeks for minor injuries, patients commonly report a reduction in pain and return to motion. For major injuries, however, patients report a reduction in pain after one year, two years, and even more time. Even after these lengthy time periods, an equally significant number of patients still report pain and no return to motion.

Healing time for more traumatic sprains and strains with rest, ice, compression, and elevation can be much longer, typically ranging from 4 to 6 months. Even then, if the injury is still unstable after this time, surgery is often required to stabilize the joint. This prolonged healing time represents a significant loss of mobility, and delay in return to functional activity. Even for the majority of sprains and strains, the current standard of care also suffers from several practical drawbacks in addition to inadequate healing. Ice can only be applied for a limited time, as prolonged contact is either not practical because it melts or causes even more discomfort and pain because of the cold temperature being applied to the affected extremity. Compression with current devices, especially with elastic wraps, is either inadequate for applying a sufficient and consistent positive force (e.g., the wrap slips over time or is applied and re-applied incorrectly), or actually restricts blood flow and lymph flow.

The dressing 100 can effectively splint and stabilize a joint, such as the knee 205. The dressing 100 can pull the tissue site 200 outwardly. This pulling force adjacent to the epidermis 300, coupled with the immobilization of the joint, can stimulate the blood flow (perfusion) and lymphatic flow at the tissue site 200, which can accelerate healing of the damaged ligament and/or muscle. Damaged tissue can be properly supplied and evacuated with blood flow and lymph flow, thereby promoting perfusion in the subcutaneous portions of the tissue site 200 and reducing edema to accelerate healing. In contrast, current treatments may only temporarily reduce inflammation by icing and may actually constrict blood flow and lymph flow by compression. Thus, the dressing 100 can provide the advantages of managing pain by reducing swelling and inflammation, increasing stability to the tissue site 200, and accelerating healing by increasing blood flow and lymph flow. In testing, about 50% increased average flow rates have been observed. In some testing, peak measurements of 70% increased air flow have been observed. Another advantage of the dressing 100 is that it can provide the benefits of opening the flow channels of the tissue site without the need for a tethered negative pressure therapy system.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some embodiments may also be omitted, combined, or replaced by alternative features serving the same, equivalent, or similar purpose without departing from the scope of the invention defined by the appended claims.

## Claims

1. An apparatus for increasing fluid flow through a tissue site, the apparatus comprising:
a first layer (105) shaped to at least partially encircle the tissue site, the first layer (105) having an unloaded state and a loaded state;
a second layer (110) coupled to the first layer (105); and
a third layer (115) comprising an adhesive coupled to the second layer (110) opposite the first layer (105), the third layer (115) being coupled to the tissue site by the adhesive;
wherein the first layer (105) comprises a biasing element which biases the first layer (105) to the unloaded state;
wherein the first layer (105) can be deflected from the unloaded state to the loaded state;
wherein the first layer (105) radially pulls on the tissue site to increase lymphatic flow through the tissue site.

2. The apparatus of claim 1, wherein the first layer (105) has an arc-shaped cross section with a central angle of at least 180 degrees when the first layer (105) is in the unloaded state.

3. The apparatus of claim 1, wherein the first layer (105) has an arc-shaped cross section with a central angle of at least 270 degrees when the first layer (105) is in the unloaded state.

4. The apparatus of any of claims 1-3, wherein the first layer (105) includes a plurality of holes.

5. The apparatus of any of claims 1-4, wherein the first layer (105) has a thickness of about 1 to about 3 millimeters.

6. The apparatus of any of claims 1-5, wherein the first layer (105) has a thickness that varies across the first layer (105).

7. The apparatus of any of claims 1-6, wherein the first layer (105) comprises polypropylene or acrylonitrile butadiene styrene (ABS).

8. The apparatus of any of claims 1-7, wherein the first layer (105) includes a first cuff (215), a second cuff (220), and a stem (225) extending therebetween.

9. The apparatus of any of claims 1-8, wherein the first layer (105) exerts a pulling force on the tissue site and wherein the third layer (115) has a bond strength at least 30% greater than the pulling force.

10. The apparatus of any of claims 1-9, wherein the second layer (110) comprises a conformable material which conforms to the shape of the tissue site.

11. The apparatus of any of claims 1-10, wherein the second layer (110) comprises open-cell foam or a closed-cell foam.

12. The apparatus of claim 11, wherein the second layer (110) includes one or more apertures.

13. The apparatus of any of claims 1 - 12, wherein the second layer (110) has a thickness of about 2 to about 4 millimeters.

14. The apparatus of any of claims 1-13, wherein the second layer (110) includes activated charcoal.

15. The apparatus of any of claims 1-14, wherein the second layer (110) is coated with oxysalts.

## Patentansprüche

1. Eine Einrichtung zum Erhöhen eines Fluidflusses durch eine Gewebestelle, die Einrichtung aufweisend:
eine erste Schicht (105), die so geformt ist, dass sie die Gewebestelle mindestens teilweise umgibt, wobei die erste Schicht (105) einen unbelasteten Zustand und einen belasteten Zustand aufweist;
eine zweite Schicht (110), die mit der ersten Schicht (105) gekoppelt ist; und
eine dritte Schicht (115), die einen Klebstoff aufweist, der mit der zweiten Schicht (110) gegenüber der ersten Schicht (105) gekoppelt ist, wobei die dritte Schicht (115) durch den Klebstoff mit der Gewebestelle gekoppelt ist;
wobei die erste Schicht (105) ein Vorspannelement aufweist, das die erste Schicht (105) in den unbelasteten Zustand vorspannt;
wobei die erste Schicht (105) von dem unbelasteten Zustand in den belasteten Zustand auslenkbar ist;
wobei die erste Schicht (105) radial an der Gewebestelle zieht, um den Lymphfluss durch die Gewebestelle zu erhöhen.

2. Die Einrichtung nach Anspruch 1, wobei die erste Schicht (105) einen bogenförmigen Querschnitt mit einem Mittelpunktswinkel von mindestens 180 Grad aufweist, wenn sich die erste Schicht (105) im unbelasteten Zustand befindet.

3. Die Einrichtung nach Anspruch 1, wobei die erste Schicht (105) einen bogenförmigen Querschnitt mit einem Mittelpunktswinkel von mindestens 270 Grad aufweist, wenn sich die erste Schicht (105) im unbelasteten Zustand befindet.

4. Die Einrichtung nach einem der Ansprüche 1 bis 3, wobei die erste Schicht (105) eine Vielzahl von Löchern aufweist.

5. Die Einrichtung nach einem der Ansprüche 1 bis 4, wobei die erste Schicht (105) eine Dicke von etwa 1 bis etwa 3 Millimetern aufweist.

6. Die Einrichtung nach einem der Ansprüche 1 bis 5, wobei die erste Schicht (105) eine Dicke aufweist, die über die erste Schicht (105) hinweg variiert.

7. Die Einrichtung nach einem der Ansprüche 1 bis 6, wobei die erste Schicht (105) Polypropylen oder Acrylnitril-Butadien-Styrol (ABS) aufweist.

8. Die Einrichtung nach einem der Ansprüche 1 bis 7, wobei die erste Schicht (105) eine erste Manschette (215), eine zweite Manschette (220) und einen sich dazwischen erstreckenden Schaft (225) aufweist.

9. Die Einrichtung nach einem der Ansprüche 1 bis 8, wobei die erste Schicht (105) eine Zugkraft auf die Gewebestelle ausübt und wobei die dritte Schicht (115) eine Bondfestigkeit aufweist, die mindestens 30 % größer ist als die Zugkraft.

10. Die Einrichtung nach einem der Ansprüche 1 bis 9, wobei die zweite Schicht (110) ein anpassungsfähiges Material aufweist, das sich der Form der Gewebestelle anpasst.

11. Die Einrichtung nach einem der Ansprüche 1 bis 10, wobei die zweite Schicht (110) einen offenzelligen Schaumstoff oder einen geschlossenzelligen Schaumstoff aufweist.

12. Die Einrichtung nach Anspruch 11, wobei die zweite Schicht (110) eine oder mehrere Öffnungen aufweist.

13. Die Einrichtung nach einem der Ansprüche 1 bis 12, wobei die zweite Schicht (110) eine Dicke von etwa 2 bis etwa 4 Millimetern aufweist.

14. Die Einrichtung nach einem der Ansprüche 1 bis 13, wobei die zweite Schicht (110) Aktivkohle enthält.

15. Die Einrichtung nach einem der Ansprüche 1 bis 14, wobei die zweite Schicht (110) mit Oxysalzen beschichtet ist.

## Revendications

1. Appareil destiné à augmenter le débit de fluide dans un site tissulaire, l'appareil comprenant :
une première couche (105) formée pour au moins encercler partiellement le site tissulaire, la première couche (105) ayant un état non chargé et un état chargé ;
une deuxième couche (110) accouplée à la première couche (105) ; et
une troisième couche (115) comprenant un adhésif accouplé à la deuxième couche (110) en face de la première couche (105), la troisième couche (115) étant accouplée au site tissulaire par l'adhésif ;
dans lequel la première couche (105) comprend un élément de polarisation qui polarise la première couche (105) à l'état déchargé ;
dans lequel la première couche (105) peut être déviée de l'état non chargé à l'état chargé ;
dans lequel la première couche (105) exerce une traction radiale sur le site tissulaire pour augmenter le flux lymphatique à travers le site tissulaire.

2. Appareil selon la revendication 1, dans lequel la première couche (105) a une section transversale en forme d'arc avec un angle central d'au moins 180 degrés lorsque la première couche (105) est à l'état déchargé.

3. Appareil selon la revendication 1, dans lequel la première couche (105) a une section transversale en forme d'arc avec un angle central d'au moins 270 degrés lorsque la première couche (105) est à l'état déchargé.

4. Appareil selon l'une quelconque des revendications 1 à 3, dans lequel la première couche (105) comporte une pluralité de trous.

5. Appareil selon l'une quelconque des revendications 1 à 4, dans lequel la première couche (105) a une épaisseur d'environ 1 à 3 millimètres.

6. Appareil selon l'une quelconque des revendications 1 à 5, dans lequel la première couche (105) a une épaisseur qui varie d'un côté à l'autre de la première couche (105).

7. Appareil selon l'une quelconque des revendications 1 à 6, dans lequel la première couche (105) comprend du polypropylène ou de l'acrylonitrile butadiène styrène (ABS).

8. Appareil selon l'une quelconque des revendications 1 à 7, dans lequel la première couche (105) comporte une première manchette (215), une seconde manchette (220) et une tige (225) qui s'étend entre les deux.

9. Appareil selon l'une quelconque des revendications 1 à 8, dans lequel la première couche (105) exerce une force de traction sur le site tissulaire et dans lequel la troisième couche (115) a une force d'adhérence supérieure d'au moins 30 % à la force de traction.

10. Appareil selon l'une quelconque des revendications 1 à 9, dans lequel la seconde couche (110) comprend un matériau conformable qui s'adapte à la forme du site tissulaire.

11. Appareil selon l'une quelconque des revendications 1 à 10, dans lequel la deuxième couche (110) comprend une mousse à cellules ouvertes ou une mousse à cellules fermées.

12. Appareil selon la revendication 11, dans lequel la deuxième couche (110) comporte une ou plusieurs ouvertures.

13. Appareil selon l'une quelconque des revendications 1 à 12, dans lequel la deuxième couche (110) a une épaisseur d'environ 2 à environ 4 millimètres.

14. Appareil selon l'une quelconque des revendications 1 à 13, dans lequel une deuxième couche (110) comporte du charbon actif.

15. Appareil selon l'une quelconque des revendications 1 à 14, dans lequel la deuxième couche (110) est recouverte d'oxysels.
